(19) Europäisches Patentamt
European Patent Office
Office européen des brevets

(11) **EP 2 812 062 B1**

(12) **EUROPEAN PATENT SPECIFICATION**

(45) Date of publication and mention
of the grant of the patent:
**31.07.2019 Bulletin 2019/31**

(51) Int Cl.:
***A61M 25/00*** *(2006.01)*      ***A61M 25/09*** *(2006.01)*
***A61B 17/22*** *(2006.01)*

(21) Application number: **13746563.9**

(22) Date of filing: **12.02.2013**

(86) International application number:
**PCT/IB2013/051144**

(87) International publication number:
**WO 2013/118105 (15.08.2013 Gazette 2013/33)**

(54) **GUIDE WIRE FOR USE WITH CARDIOVASCULAR LESIONS**

FÜHRUNGSDRAHT ZUR VERWENDUNG FÜR KARDIOVASKULÄRE LÄSIONEN

FIL-GUIDE DESTINÉ À ÊTRE UTILISÉ POUR DES LÉSIONS CARDIOVASCULAIRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.02.2012 US 201261597783 P**

(43) Date of publication of application:
**17.12.2014 Bulletin 2014/51**

(73) Proprietor: **Microbot Medical Ltd.
Caesarea 3079861 (IL)**

(72) Inventors:
• **SHEKALIM, Avraham
36842 Nesher (IL)**
• **PELEG, Noam
19351 Gan Ner (IL)**

(74) Representative: **AWA Denmark A/S
Strandgade 56
1401 Copenhagen K (DK)**

(56) References cited:
WO-A2-03/018085         US-A- 5 605 162
US-A- 6 113 557          US-A1- 2009 312 747
US-A1- 2010 087 780      US-B2- 7 481 778
US-B2- 7 491 224

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

CROSS REFERENCES TO RELATED APPLICATIONS

This application claims priority from U.S. Provisional Application Serial No. 61/597,783, entitled: A GUIDE WIRE FOR CROSSING THROUGH A CARDIOVASCULAR LESION, filed on February 12, 2012.

TECHNICAL FIELD

The disclosed subject matter is in the field of cardiovascular medical devices.

BACKGROUND

In a PCI (Percutaneous Coronary Intervention) procedure, balloon angioplasty and stents are used to expand a lumen through a stenosed segment of a coronary vessel. A guide wire usually with a shaft of 0.36 mm diameter (0.014" diameter) is inserted through the lesion and the tools used to expand the vessel are positioned by sliding over the guide wire. The physician typically bends the tip of the guide wire before the insertion in order to facilitate an insertion to side branches of the main coronary vessel. The distal end segment of the guide wire is floppy (bendable, flaccid or relaxed) in order to pass through the tortuous geometry of the coronary vessels without damage.

There is a difficulty in crossing calcified segments like the proximal cap of a CTO (chronic total occlusion) with a floppy guide wire. The conventional procedure for doing so is to advance a PTCA balloon or a dedicated microcatheter over the floppy bendable guide wire. The microcatheter increase the stiffness, and accordingly increases the shaft support and the tip load of the guide wire. According to "Percutaneous Recanalization of Coronary Chronic Total Occlusions: Current Devices and Specialized Wire Crossing Techniques, Hee-Yeol Kim (Korean Circ J 2010; 40:209-215), the stiffness of the tip of the guide wire increases the closer it is to the microcatheter. However, just a short distance of a few millimeters away from the microcatheter tip, the guide wire maintains its original bendability (flaccidity or floppyness).

The microcatheter also allows the bendable floppy guide wire to be exchanged for a stiffer guide wire. The diameter of the microcatheter is relatively large compared to that of the guide wire, resulting in an inability to advance the microcatheter through heavy calcified occlusions.

Although, stiff guide wires, such as the Asahi Conquest® guide wires, allow the physician to apply sufficient power to penetrate calcified occlusions, there is an increased risk of dissecting and perforating the coronary vessel walls. The stiffness of these conventional guide wires limits their ability to be maneuvered and steered.

Therefore in order to use the aforementioned conventional guide wires in calcified occlusions, information about the lesion morphology should be obtained by use of contrast media, x-rays, and Intravascular Ultrasound (IVUS) catheters. Additionally, only highly trained cardiologists should use of these conventional guide wires. During the operation the physician may exchange several guide wires in order to use the proper wire for the requisite situation.

U.S. Patent No. US 5,040,543 to Badera, et al. discloses guide wires with movable cores. The guide wires are constructed of a movable core wire slideably received within an external shaft. The external shaft is made of a helical wire coil. The tip of the external shaft is curved to a shape of a "J" in order facilitate insertion to coronary vessels and branches thereof. The introduction of the tip of the core, into to the curved segment of the wire, straightens the shape of the core, and changes the configuration of the wire from soft to stiff. The stiffness makes the guide wire relatively easy to push through calcified occlusions. However, since the core and the external shaft are not connected the ability to transmit torque and the ability to control the tip while it is in a stiff configuration is limited, when compared to that of a conventional stiff guide wire.

U.S. Patent No. 5,605,162 to Mirzaee, et al. discloses a guide wire with a helical compression spring tip and movable core, which contracts and retracts the spring, such that the wire stiffness is adjustable. However, as the core is fixed to the tip the wire, bendability of the guide wire is poor when compared to that of guide wires without cores.

Using conventional CTO special guide wires, the physician lacks the ability to control the stiffness and tip curvature of the guide wire without using an additional sleeve, for example, a microcatheter that is over the guide wire. Controlling guide wires by microcatheters is inaccurate, as the microcatheter must be manipulated from the proximal end of the guide wire, which is held by the physician. This manipulation increases the risk of causing injuries, including perforations to the coronary vessel walls.

In addition, moving the tip back and forth or applying penetration forces to the coronary occlusion (i.e., on the proximal cap of the CTO lesion) is conventionally performed by a physician manipulating the proximal end of the guide wire. Due to the distance between the physician's hands and the site of treatment in the coronary vessels (about 0.7 to 1.5 meters), the ability of the physician to control the distal tip of the guide wire is limited. This increases the risk of causing injuries to the coronary vessel walls and reduces the ability to apply significant power for penetrating coronary lesions.

Additionally, in conventional CTO recanalization procedures, the physician introduces a floppy, soft tip guide wire- first to negotiate the lesion and then to switch the guide wire with increasingly stiffer wires. Switching wires takes substantial time. During this time, the patient, as well as the physician, are exposed to x-ray radiation and contrast media.

SUMMARY

The present disclosed subject matter improves on the safety and efficiency of conventional guide wires and procedures for their use. The present invention provides a guide wire, whose flexibility/stiffness is adjustable. By adjusting the flexibility/stiffness of the guide wire, the guide wire is steerable in very small or fine movements, allowing for precise maneuvering of the guide wire, which is crucial when advancing the guide wire through the tortuous geometry of the coronary vessels. Additionally, the disclosed guide wire operates at forces sufficient to safely penetrate and pass through, or cross, a cardiovascular lesion, such as calcified segments in coronary vessels, without causing damage to the cardiac vessels.

There is disclosed a guide wire for passing through, or crossing, cardiovascular lesions, such as calcified segments in coronary vessels. The guide wire is manually adjustable by the practitioner from extremely bendable or floppy, to stiff, of little bendability, to accommodate the environment of the coronary vessel in which the guide wire is being advanced therethrough. The guide wire is also manually steerable by the practitioner. The steerability is achieved by adjusting the curvature of the distal end, and typically, the tip, of the guide wire. As a result of this structure, during a PCI procedure, the physician can adjust the guide wire's flexibility from floppy to stiff while steering the guide wire. The tip of the guide wire can also be manually moved back and forth in the coronary vessel. All movements of the guide wire, and the tip, as well as the steering thereof are made by a hand held manual control unit.

The disclosed guide wire comprises an internal shaft slideably received, and telescopically moveable, within an external shaft. The distal portion of the external shift is made of a high elasticity coiled wire. Portions of the coil turns are spaced apart, or pitched, from each other, such that these turns form a compression spring. In one embodiment, the external shaft is made entirely of a coiled wire where the tip is made of a conically tapered turns. An elastic sleeve over the portion of closely spaced apart turns prevents retraction of these turns while the greater spaced apart or distanced turns are free to contract and retract. The internal shaft is made of a high elasticity, monofilament wire. The distal tip of the internal shaft is conically tapered.

Before insertion, the tip of the guide wire is typically bent by the physician. It is introduced to the coronary vessels through a guide catheter, through the femoral or the radial artery. While the guide wire is initially curved, and while it is advanced through the coronary arteries toward the coronary lesion of interest, the internal shaft tip is located proximally, away from the external shaft tip, leaving the tip is floppy or bendable, allowing the guide wire to pass through the coronary vessels without damaging them. Should a stiffer wire be desired at the distal end and at or near the tip of the external shaft, the inner shaft is manually advanced distally to a location at or near the tip. Such stiffness is achieved by the guide wire structure itself, without any additional operations such as introducing microcatheters and or replacing or augmenting existing shafts with stiffer wires.

In order to achieve addition control over the guide wire, the inner shaft can be advanced up to point where the outer shaft is tapered conically. The inner shaft may interlock with the tip or other structure at or proximate to the conical tapered portion of the outer shaft. The interlocking is temporary. The outer shaft is formed of coils, to impart behavior of a spring to it. The inner shaft and tip interlock at a predetermined engagement force, a force arising from a frictional engagement. When in this interlocking position, pulling of the inner shaft backward or proximally with a force weaker than the predetermined holding force, the physician can pull back or push the distal tip of the guide wire such that it is steerable through the lesion. Pulling or pushing the tip by the internal shaft is substantially more accurate and efficient than operating the guide wire by manipulating its end portion. Moreover, maneuvering the tip by the inner shaft, eliminates long uncontrolled advances of the guide wire, which may damage the coronary vessels.

The disclosed guide wire is extremely maneuverable, and the tip is such that should it be introduced into a false lumen, the tip can be redirected a the true lumen by being pulled back or retracted by the practitioner. This is a significant improvement over a guide wire which is manually manipulated from the proximal end of the wire itself, or from the introducing of a dedicated redirecting catheter.

The disclosed guide wire is also such that retracting the internal shaft with a force to overcome the engagement force, causes the internal shaft to disengage from the tip or other engagement location at the distal end of the guide wire. Upon disengagement, the tip will advance distally toward a targeted location, for example, a calcified lesion, and exert an impact force on the lesion which is at least approximately equal to the engagement force.

The disclosed guide wire is such that the tip is controlled in an accurate manner from within the inner parts of the wire. The tip is manipulated by a simple manually operated handle located at the proximal end of the wire. The tip moves without moving the wire portion that is proximal to the tip from its location relative to the vessel. This is due to an inner shaft that moves along a path defined by the external shaft of the guide wire. This handle allows the physician to modify the stiffness of the guide wire proximate to the tip and the curvature of the tip. Also, the handle allows the tip to be moved back and forth (proximally and distally), to produce significant penetration power for penetrating and passing through coronary vessel lesions. The aforementioned manipulations are performed without moving the proximal end of the guide wire from its position.

In a construction, the external and internal shafts are connected via a handle at their proximal ends. Adjustments to the engagement forces are made by rotating a knob on the handle.

In a construction, after the lesion is been penetrated and passed through by the tip of the guide wire, the practitioner can cut the proximal handle away, allowing for the introduction of catheters and other instruments over the wire, as well as other tools such as Percutaneous Transluminal Coronary Angioplasty (PTCA) balloons or a stent for the lesion.

The disclosed guide wire provides trackability and maneuverability for precise navigation and fine movements of the guide wire through the tortious geometry of the coronary vessels. The disclosed guide wire also provides stiffness, for which the aforementioned precise maneuverability and navigation can be made, while providing safe penetration forces, to impact and pass through cardiovascular lesions, while minimizing the need for additional tools. The disclosed guide wire also provides efficient and safe techniques for facilitating the installation of a PTCA balloons and stents in CTO lesions.

The disclosed guide wire has an inner shaft received within an external shaft. The distal portion of the external shift is made of a high elasticity coiled wire. The coil turns are distanced, or pitched, one from another to form a compression spring. Advancing the inner shaft towards the distal tip of the guide wire increases the tip load (stiffness) of the guide wire without additional operations such as introducing microcatheters, or replacing the wire. The distal tip of the internal shaft and the distal tip of the external shaft can be engaged in an adjustable predetermined holding force. By pulling the internal shaft relative to the external shaft the wire can be steered. By pulling the internal shaft with a force stronger than the holding force the tip of the external shaft abruptly released to produce predetermined impact forces. Successive impacts can be created in this manner to penetrate and pass through lesions, without damaging the coronary vessels.

The embodiment of the invention is directed to a guide wire. The guide wire includes a tubular member (e.g., an external shaft) including a proximal end and a distal end, the distal end including an elastic portion; a tip in communication with the distal end of the tubular member; and an inner shaft extending in the tubular member. The inner shaft moves between an engaged position, where the inner shaft is in a removable engagement with the tip, and a retracted position, where the inner shaft has been disengaged from the tip once retraction of the inner shaft from the tip reaches a predetermined retraction force. The disengagement causes the tip to move distally with a force corresponding to the predetermined force and the elasticity of the elastic portion of the tubular member. The guide wire is such that the elastic portion includes a coiled wire portion, which is arranged as a spring, and the coiled wire portion is arranged along the guide wire to allow the tip to move proximally and distally without moving the entire length of the guide wire.

BRIEF DESCRIPTION OF THE DRAWINGS

Attention is now directed to the drawing figures where like reference numerals or characters refer to corresponding or like components. The drawing figures are as follows.

FIG. 1 illustrates a preferred embodiment of the innovation, including a manual control handle located at the proximal side of a wire having a controlled tip.

FIG. 2 is a schematic cross section of the distal portion including the tip of the wire of FIG. 1.

FIG. 3 is a schematic cross section of the wire of FIG. 2 where its inner shaft is advanced toward the tip member.

FIG. 4 shows the interlocking interface between the inner shaft tip and the tip member of the external shaft.

FIG. 4A illustrates the forces acting on the tip member interface where there is interlocking (engaging) with the inner shaft from FIG 4.

FIG. 5 is a schematic cross section of the manual control handle of FIG. 1.

FIG. 6 is a schematic cross section of the wire of FIG. 2 where it inner shaft is interlocked with the tip member.

FIG. 7 is a schematic cross section of the wire of FIG. 2 where it inner shaft is released from it interlock (engagement) with the tip member.

FIG. 8 is a schematic cross section in an embodiment of the tip of the wire of FIG. 2 where, the tip member is a tapered portion of the external shaft coil.

FIG. 9 is a schematic cross section in the tip of the wire of FIG. 8 where, the inner shaft is interlocked with the tapered portion of the external shaft coil.

FIG. 10 is a schematic cross section of an embodiment of a guide wire.

FIGs. 11, 11A, 11B-1 to 11B-3, and 11C-1 to 11C-6 are schematic cross sections of an embodiment of the guide wire with a tip including an inner core wire for interlocking and engaging with a tubular inner shaft.

FIG. 11D is a diagram of a handle for use with the guide wires shown in FIGs. 11, 11A, 11B-1 to 11B-3, and 11C-1 to 11C-6.

FIGs. 12 and 12A are schematic cross sections of an embodiment where an inner shaft includes distal protrusions for facilitating interlocking (engaging) of the inner shaft with a tip member.

FIGs. 13 and 13A are schematic cross sections of an embodiment of a guide wire having a safety wire.

FIGs. 14A to 14C are schematic cross sections of an embodiment of a guide wire having a slide interlock feature.

FIGs. 15A and 15B are schematic cross sections of an embodiment of a guide wire having a proximal interlock feature.

FIG. 16 is a schematic cross section of an embodiment of a guide wire having an external telescopic tube arrangement.

FIGs. 17A to 17C are schematic cross sections of an embodiment of a guide wire having a safety feature.

FIGs. 18A and 18B are the guide wire of FIGs. 14A to 14C, with radio-opaque markers.

FIG. 19A is a perspective view of a guide wire with a torqueing feature.

FIGs. 19B and 19C are schematic cross sections of the guide wire of FIG. 19A.

FIG. 19D is a cross-section of the guide wire taken along line Y-Y of FIG. 19C.

DETAILED DESCRIPTION OF THE DRAWINGS

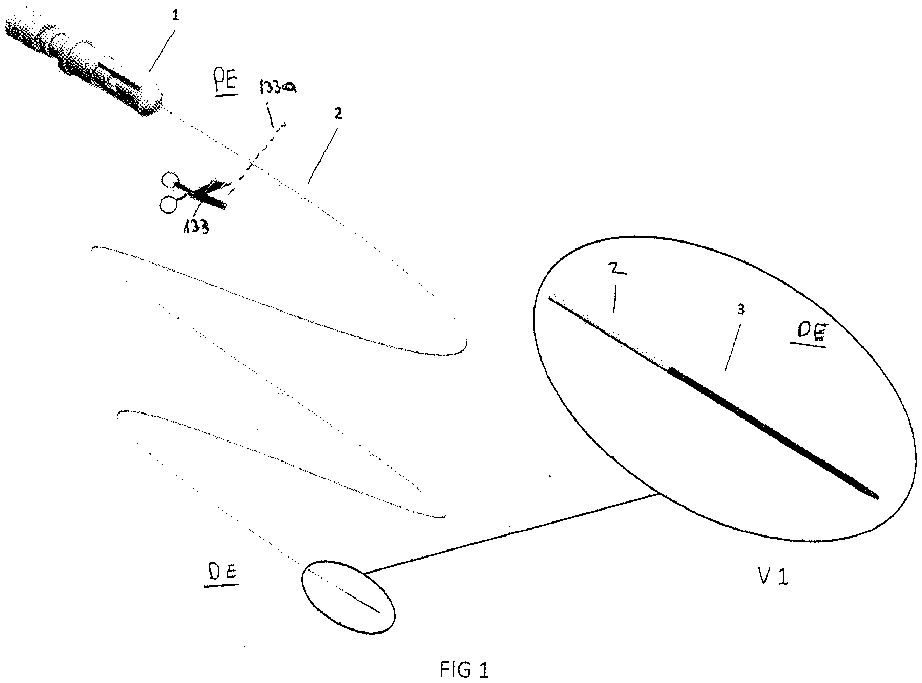

An embodiment of the invention is illustrated in FIG 1. A manual control handle 1 is located at the proximal end PE of a guide wire (wire) 2, for example, a 0.014 inch (0.03556 cm) diameter guide wire, having a controllable tip 3. The guide wires 2, 2-1010, 2-1011, 2-1012, 2-1013, 2-1014, 2-1015, 2-1016, 2-1017 and 2-1019, as disclosed herein, are for example, 0.014 inches (0.03556 cm) in diameter. The guide wire 2, as well as the aforementioned guide wires disclosed herein, include a proximal end PE and a distal end DE. Throughout the description of the embodiments of the invention herein, the terms proximal and distal are directional and orientational, and the directions and orientations referenced by these terms are in accordance with the proximal end PE and distal end DE of the guide wire 2 (as shown in FIG . 1). The tip 3, at the distal end DE of the guide wire 2, is further illustrated in the detailed view V1 of FIG. 1, is designed for impacting and safely passing through lesions in coronary vessels. Movement of the tip 3 is manually generated and controlled, as detailed further below.

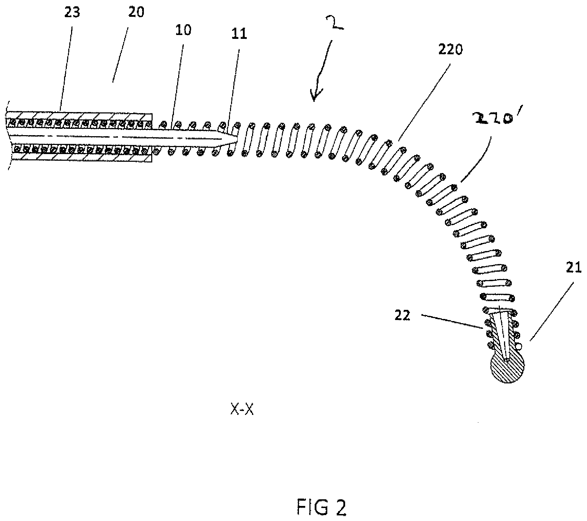

As illustrated in FIG 2, the guide wire 2 includes an inner (internal) shaft 10, which is slideably received within an external (outer) shaft 20. The shafts 10, 20, including all inner shafts and external shafts for the guide wires disclosed herein, are made, for example, of high elasticity interventional medical grade materials such as medical grade metals and polymers, stainless steel spring, platinum or Nitinol, and the like, and combinations thereof, as tubes and wires.

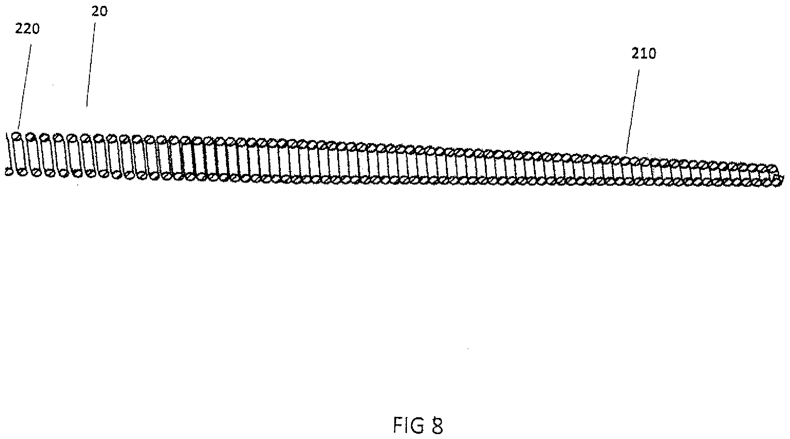

The inner shaft 10, shown in FIGs. 2, 3, 4, 5, 6 and 7, is, for example, a wire, tapered at its distal end (the inner shaft 10 having the same proximal/distal orientation as the wire 2), which forms a tip 11. The external shaft 20 includes a coiled wire 220, including a distal coiled wire portion 22, which receives a tip member 21 . Turning also to FIG. 8, in the embodiment illustrated therein, the distal end of the external shaft 20 (the external shaft 20 having the same proximal/distal orientation as the wire 2) is coiled conically to form a tapered tip 210. The coils of the coiled wire portion 22 at the tip member 21 are spaced apart (coiled) from each other such that a helical compression spring is formed. The coils of the coiled wire 220 in the portion 220' proximal to the tip member 21, are, for example, pitched at a wider distance apart than those of the distal coiled wire portion 22. For example, the turns of the coiled wire 22, which are proximal to the pitched coiled wire 220 are covered by a thin polymeric sleeve 23, which is fixed to and prevents retraction of the coiled wire 220.

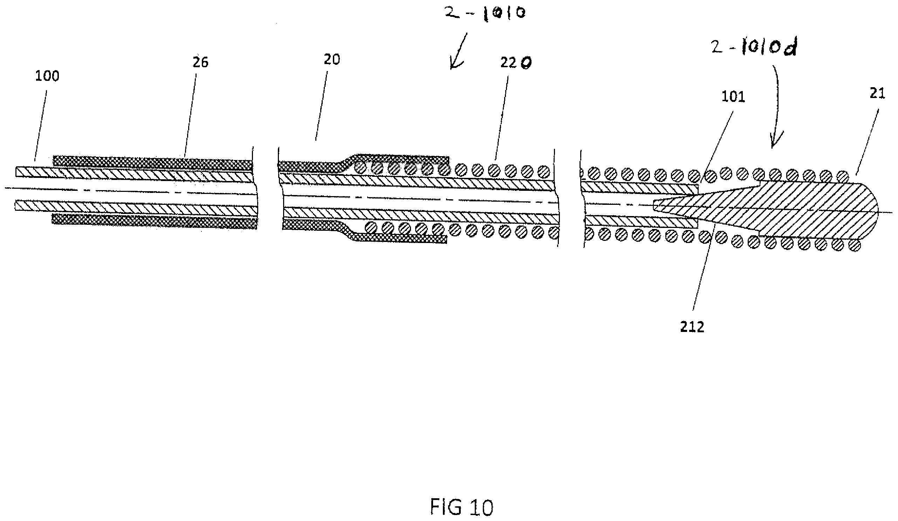

In alternative embodiments, for example, as shown by the wires 2-1010, 2-1011, and 2-1012, in FIGs. 10, 11, 11A, 12 and 12A, respectively (having the same proximal end/distal end orientation as the wire 2 of FIG. 1 above), the distal portion 2-1010d, 2-1011d and 2-1012d, e.g., approximately 330 nm to 100 mm, of the external shaft 20 is formed by the coiled wire 220, in accordance with the description above. The proximal portion of the external shaft 20 is formed of a tube 26, for example, a polymeric or high elastomeric interventional medical grade materials, such as stainless steel spring or Nitinol. In these wires 2-1010, 2-1011 and 2-1012, the external shaft 20/26 is flexible, and can transmit torque applied to it to the tip member 21. By transmitting torque, this structural arrangement allows for maneuverability of the wires 2-1010, 2-1011 and 2-1012 to make fine and precise movements, to accommodate and operate safely in the tortious geometry of the coronary vessels, without damaging the coronary vessels, including perforating them.

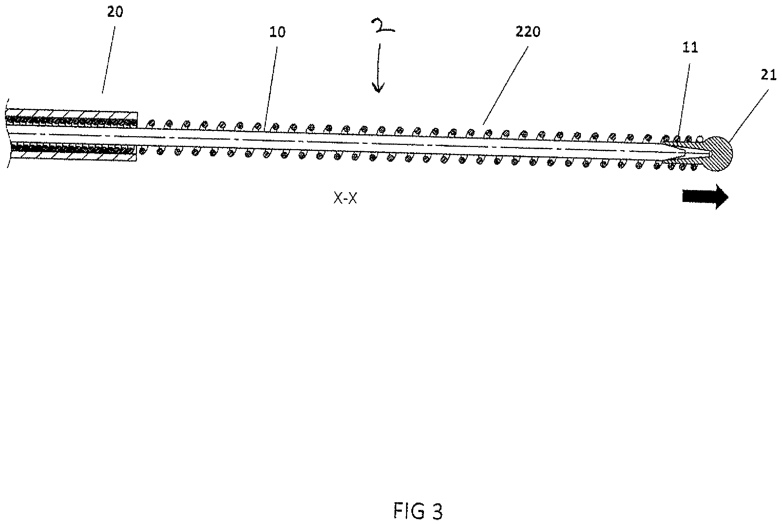

Turning back to FIG 2, while advancing the guide wire 2 towards the segment of interest, the distal end 11 of the inner shaft 10 is proximally away of the tip 21 of the external shaft 20. In the orientation as shown, the tip member 21 of the wire 2 is floppy, and as such, can be advanced toward a lesion without the risk of causing damage, such as dissections or perforation of the coronary vessel. Additionally, advancing the inner shaft 10 (distally) towards the tip member 21, as illustrated in FIG 3, increases the "tip load" (e.g., stiffness) of the tip member 21 of the wire 2 without additional operations, such as introducing a microcatheter over the wire 2 or replacing the wire 2 with a stiffer wire.

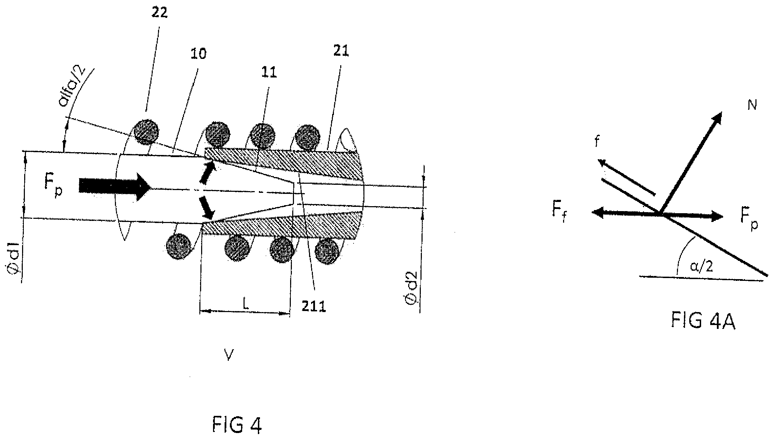

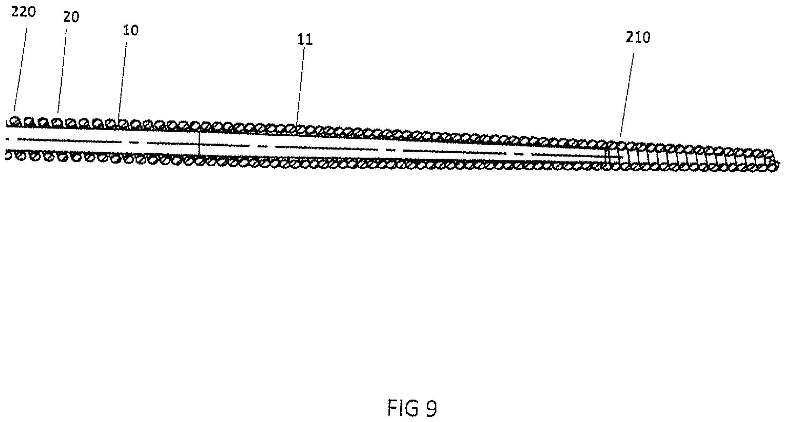

As illustrated in FIG. 4, by pushing the inner shaft 10 against the tip member 21, the distal tapered tip 11 is engaged in a narrowing lumen 211 in the tip member 21 of the wire 2. Alternatively, as illustrated in FIG 9, the inner shaft 10 is engaged by the narrowing geometry of the conically tapered coil portion of the external shaft tip 210 (of the external shaft 20). The holding (engaging) force engaging the inner shafts 10 in the narrowing lumen 211 and external shaft tip 210, respectively, is in direct relation to the pushing (distally directed) forces been applied to by the inner shafts 10 (and the tips 11 of the inner shafts 10) to the respective narrowing limen 211 (FIG. 4) and external shaft tip 210.

In another embodiment that is illustrated in the wire 2-1010 of FIG. 10, the inner shaft 100 is hollow. The distal end 101 of this inner shaft 100 can interlock (e.g., temporarily frictionally engage) with the conically tapered end 212 of the tip member 21 (defining the tip of the guide wire 2-1010, in accordance with the tip 3 of the guide wire 2 of FIG. 1, and described above). The blunt shape of the tip 101 of the inner shaft 100 minimizes the risk of the inner shaft 100 passing through the coiled wire 220.

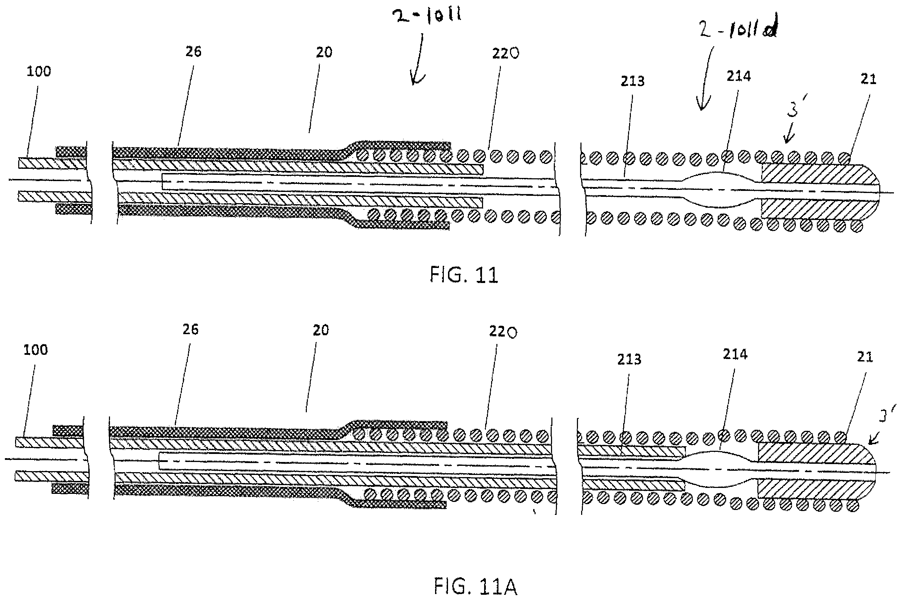

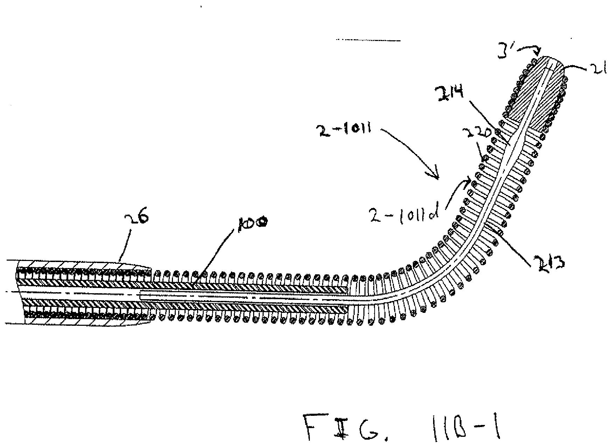

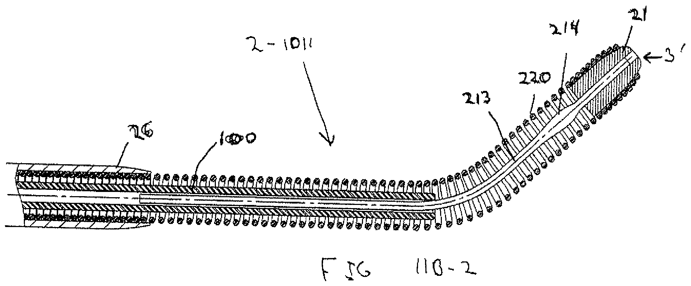

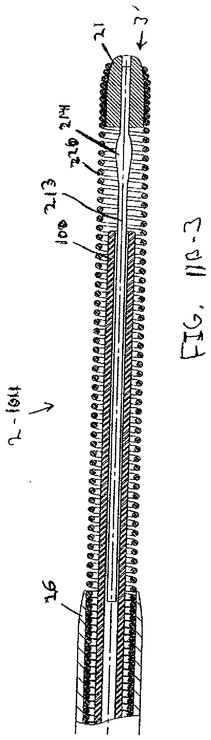

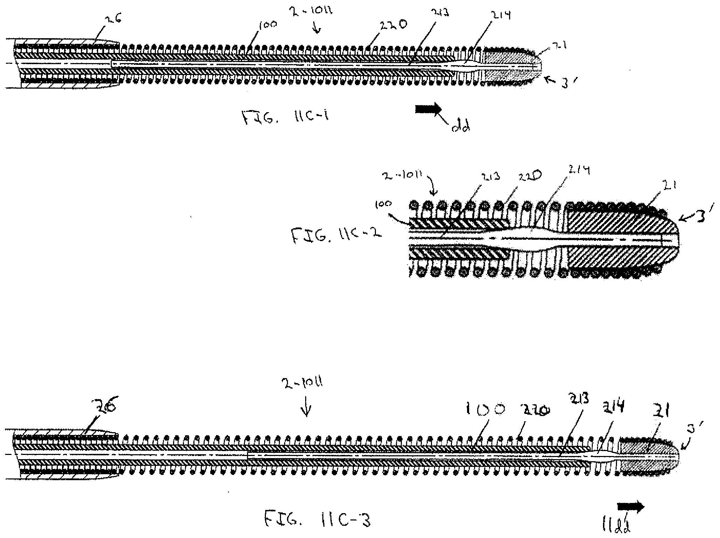

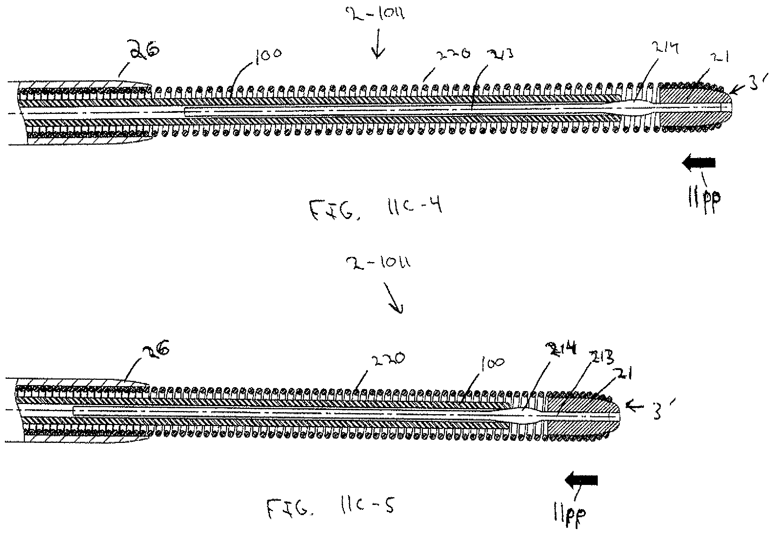

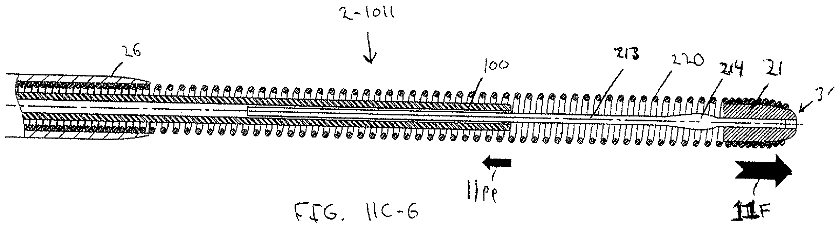

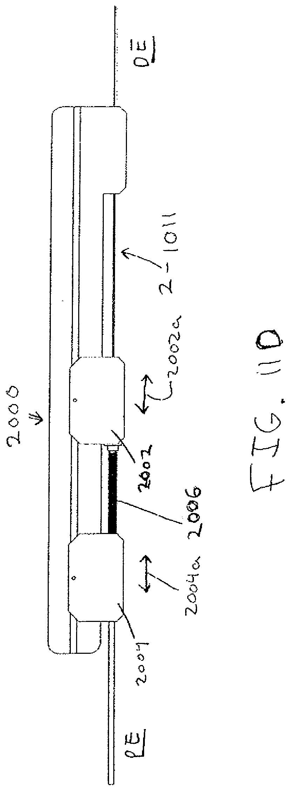

As illustrated in FIGs. 11 and 11A, a thin core wire 213 is connected in a fixed manner to the tip member 21. The tip member 21 and the core wire 213, which is fixed in the tip member 21, define the tip 3' of the guide wire 2-1011, in accordance with the tip 3 of the guide wire 2 of FIG. 1, and described above. This structural arrangement allows the inner shaft 100 to slide over the core wire 213 in a telescoping manner, into frictional contact with a tapered thickened protrusion 214 (with a larger diameter than the core wire 213 and the inner shaft 100), causing a frictional engagement between the core wire 213 and the inner shaft 100, as illustrated in FIG. 11A. The thinness of the core wire 213 allows the inner shaft 100 to engage (e.g., in a frictional engagement) with the tip member 21, even a tip member 21 with a sharp curved geometry. This orientation of components also allows the physician to shape the wire tip 3' according to specific needs. The guide wire 2-1011 is also such that the inner shaft 100 is torqueable at a proximal end and the inner shaft 100 transmits torque to the tip 3' upon rotating the core wire 213 during proximal and distal telescopic movement (and also while the tip 3 and inner shaft 100 are engaged).

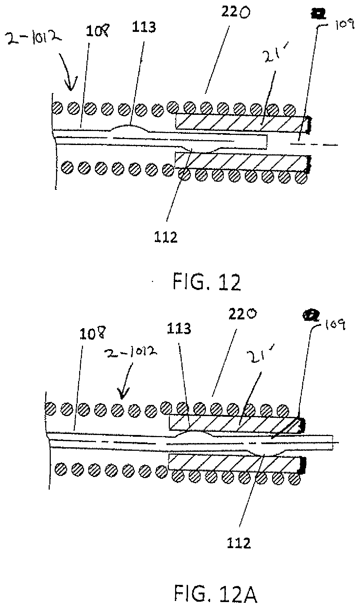

Another alternative structural arrangement for a releasable interlock (temporary engagement, including a temporary frictional engagement) of the inner shaft 108 to the tip member (not shown) is illustrated in FIGs. 12 and 12A. The inner shaft 108, for example, is a wire, and includes two protrusions 112, 113, extending in opposite directions, proximal and distal, and are spaced apart from each other on the inner shaft 108, at the distal portion of the inner shaft 108. This protrusion 112, 113 structure allows for easy insertion of the inner shaft 108 into the channel 109 of the tip member 21' (defining a tip of the guide wire 2-1012, in accordance with the tip 3 of the guide wire 2 of FIG. 1, and described above). The advancement of the inner shaft 108 into a position where both protrusions 112, 113, engage the tip member 21' results in a clamping between the tip member 21' and the inner shaft 108 (FIG. 12A), whereby a fixed predetermined force (e.g., frictional force) is required to release the engagement.

In FIG 4A, there is shown a diagram of the forces acting at on engagement (or interlocking) between the inner shaft 10 at its tip 11 and the tip member 21. Also, with reference to FIGs. 2, 3, 6 and 7, the tapered tip 11 of the inner shaft 10 is pushed in an axial force Fp or distally towards the wall 211 of the narrowing lumen of the tapered tip member 21. Assuming the lumen wall 211 and the tip 11 are laniary elastic (i.e. no plastic stress and strains are exists), the friction force f between the tip 11 and the wall 211 (of the tip member 21) is equal to the normal force N on the wall multiply by the friction coefficient $\mu$ between the parts ($\mu$ =0.1 to 0.15 for friction between two steel faces).

The axial component of the friction force $F_f$ is:

$$F_f = \mu \frac{F_p}{\tan\left(\frac{\alpha}{2}\right)}$$ where $\alpha$ is the angle of the walls

211g of the tip member 21. The engaging or interlocking effect is expressed as: $$\mu \geq \tan\left(\frac{\alpha}{2}\right)$$

or $$\mu \geq \left(\frac{d_1 - d_2}{2L}\right)$$ . Because the

elasticity of the inner shaft 10 and tip member 21 causes static friction force between these structures, a normal or engaging force remains on these components after the engaging or pushing force is removed, such that the inner shaft 10 and tip member 21 remain engaged (in a temporary engagement until the engagement force is removed, as detailed below). To release the engagement or interlock, the user must pull the inner shaft 11 proximally with a force stronger than $F_f$. Therefore, $F_f$ is the holding force between the tip 11 of the inner shaft 10 and the tip member 21.

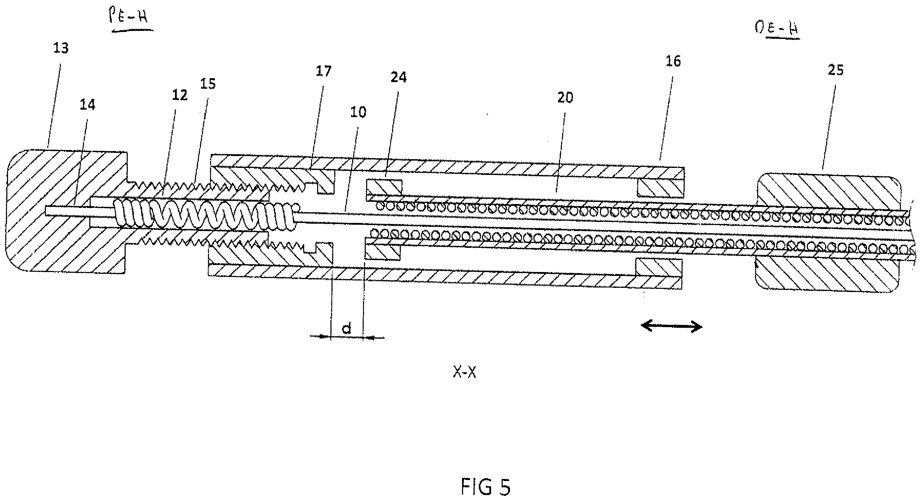

Turning to FIG. 5, there is illustrated the control handle 1. At the proximal end PE-H of the handle 1, the inner shaft 10 is attached to a spring 12. In other embodiments, the proximal end PE-H of the inner shaft 10 can be coiled to form a spring. The end of the proximal spring 14 is connected to a knob 13, the knob 13 connected by a screw thread 15 to a sliding member 16. The knob 13 allows axial movement, both proximally and distally, of the inner shaft 10. The sliding member 16 can slide axially along the external shaft 20. The motion of the sliding member 16 is limited by the stopper 24, abutting stop members 24p (in the direction of the proximal handle end PE-H) and 24d (in the direction of the distal handle end DE-H). Another gripping collar 25 is fixed to the external shaft 20, and it allows the physician to grip and move axially, both distally and proximally, the external shaft 20.

Movement of the knob 13 allows for fine, small and precise movements, of the inner shaft 10, while movement of the sliding member 16 allows for both small and fine movements, and also large or coarse movements of the inner shaft 10. The primary movements and navigations of the inner shaft 10, and the inner shafts disclosed herein, both proximally and distally, are typically accomplished by moving the sliding member 16, while secondary movements of the inner shaft 10, and the inner shafts disclosed herein, both proximally and distally, are performed by turning the knob 13. Movement of the gripping member 25 allows for movements, typically long or coarse movements, of the external shaft 20.

The position of the structural components illustrated in FIG. 5 corresponds with the position of the tip 11 of the inner shaft 10, illustrated in FIG. 3, where the tip 11 of the internal shaft 10 is proximate to the cavity in the tip member 21 of the external shaft 20. The distance "d," between the proximal stop member 24p (which also serves as the distal end of the threaded sleeve 17 of the sliding member 16), is predetermined by the screw treads 15.

When the sliding member 16 is in its proximal position, next to the stopper 24, the tip 11 of the inner shaft 10 is out of contact and out of engagement with the tip member 21 of the external shaft 20, as illustrated in FIG. 2. Accordingly, the wire 2 is floppy or flaccid. By pushing the sliding member 16 to the position illustrated in FIG. 3 and FIG. 5, the tip 11 of the inner shaft 10 is advanced distally, toward tip member 21 of the guide wire 2, increasing the stiffness of the wire 2 without any additional operations, devices or instruments.

By pushing the sliding member 16 distally, toward the stopper 24, for a distance "d," the tip 11 of the inner shaft 10 engages the tip member 21 of external shaft 20, resulting in an interlocking or engagement of these structures, as illustrated in FIG 4. The interlocking or engagement force is produced by the compression of the proximal spring 12 (FIG. 5). Because of the conical tapered shape of the tip member 21, the holding or engaging force between the tip 11 and tip member 21 is determined according to the force produced by the spring 12, and according to the angle of the tapered shapes. Accordingly, holding or engaging force is predetermined by the distance "d," which in turn, can be adjusted by rotating the knob 13 relative to the sliding member 16.

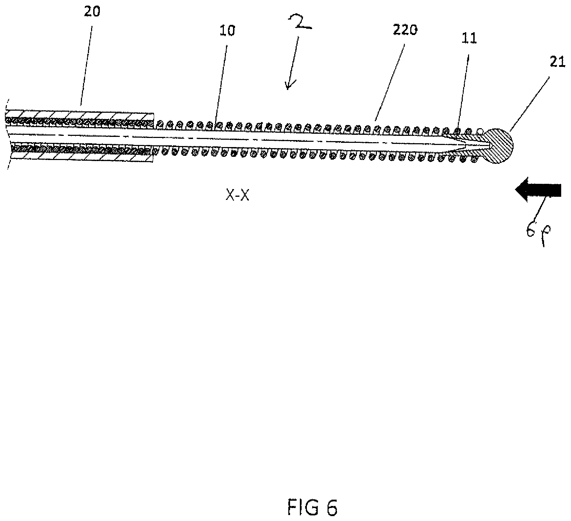

With the handle 1 in the aforementioned position (FIG. 5) and the wire 2, as shown in FIG. 3, pulling the sliding member 16 rearward (i.e., moving the sliding member 16 proximally, relative to the external shaft 20), pulls the inner shaft 10 proximally, which as illustrated in FIG. 6, in particular, the arrow 6p, cocks or loads the spring portion 220 of the wire 2.

Pulling (or pushing) the inner shaft 10 backward (proximally) or forward (distally) while the inner shaft 10 is engaged with the tip member 21 allows the physician to precisely position the tip 3 of the guide wire within the vessel or the lesion therein, while the proximal portion of the wire 2 remains in place. As a result, the tip 3 can be redirected or maneuvered backward (proximally) or forward (distally) to precisely navigate the lesion.

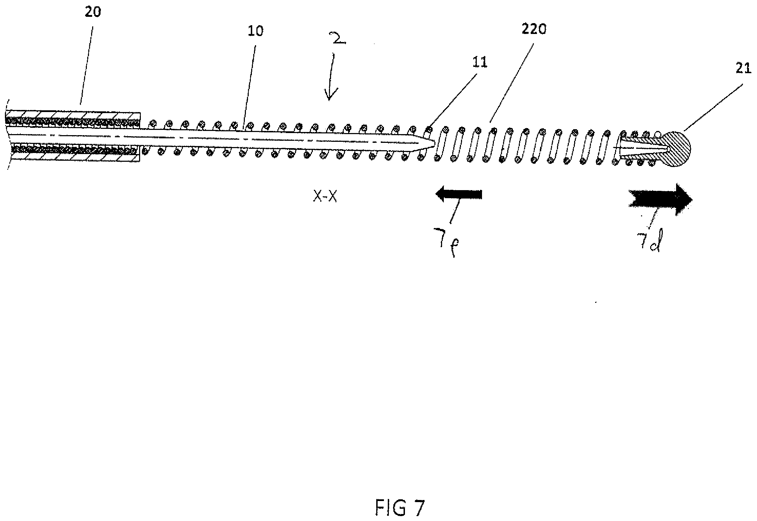

If the pulling force (illustrated by the arrow 7p) is larger than the predetermined holding force, the engagement of the inner shaft 10 in the tip member 21 abruptly release the tip 11 of the inner shaft 10 from the engagement with the tip member 21, as shown in FIG. 6. As shown in FIG. 7, the tip member 21 of the external shaft 20 will move forward (distally), as illustrated by the arrow 7d, with an impact force equal to the holding or engagement force, which just been released. The wire 2 has sufficient force to penetrate and pass through calcified segments.

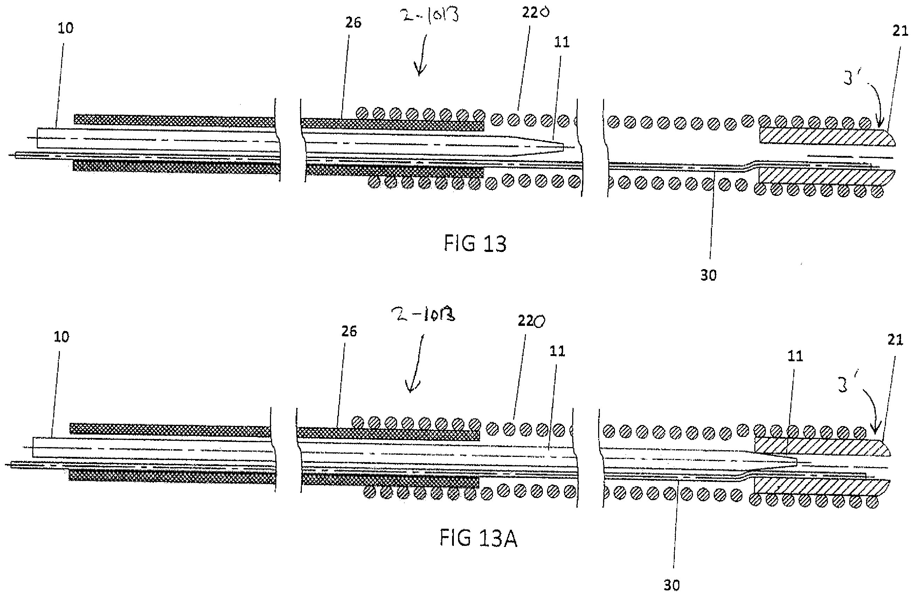

Attention is now directed to FIGs. 11B-1, 11B-2 and 11B-3, to illustrate the telescopic structure and maneuverability and steering aspects of the guide wire 2-1011 in detail. These structure detailed is also representative of the telescopic structure and maneuverability and steering of guide wires 2-1010 (FIG. 10) and 2-1013 (FIGs. 13 and 13A). In FIG. 11B-1, the guide wire 2-1011 is in an initial state at and initial curvature.

As the inner shaft 100 is advanced distally, toward the tip member 21, as described above for FIG. 5, for example, by moving the sliding member 16 distally, for large or coarse movements, as shown in FIG. 11B-2, the curvature and stiffness of the guide wire 2-1011 is adjusted. Specifically, such the inner shaft 100 has moved in a telescopic manner over the inner core wire 213 Here, the stiffness of the guide wire 2-1011 is increased at the distal end of the guide wire 2-1011 and the guide wire 2-1011 curvature is decreasing as the guide wire 2-1011 it is straightening. Alternately, should small or fine movements, both proximally and distally, for precision steering of the inner shaft 100 and guide wire 2-1011 be desired, the knob 13 can be turned accordingly. The distal movement of the inner shaft 100 continues until the guide wire 2-1011 has straightened, as shown in FIG. 11B-3. The guide wire 2-1011 is now in a stiff state.

Attention is now directed to FIGs. 11C-1 to 11C-6, to illustrate the engagement and impact forces for the guide wire 2-1011, with telescopingly arranged elements. The structure detailed is also representative of the engagement and impact forces of guide wires 2-1010 (FIG. 10), 2-1012 (FIGs. 12 and 12A), and 2-1013 (FIGs. 13 and 13A). In FIG. 11C-1, the guide wire 2-1011 is in a stiff state, where the inner shaft 100 has been moved distally (represented by the arrow 11dd) such that there is a frictional engagement between the inner shaft 100 and core wire 213 of the tip element, at the thickened portion 214. The engagement causes a frictional retention of the inner shaft 100 with the core wire 213 of the tip element, as shown in detail in FIGs. 11C-2 and 11C-3. FIGs. 11C-2 and 11C-3 show the temporary attachment of the inner shaft 100 to the tip member 21(via the core wire 213) allows for precise navigation motion of the tip member 21 without moving the entire length of the guide wire 2-1011.

In FIG. 11C-4, a pulling force for retraction of the inner shaft 100, in the proximal direction (of arrow 11pp), has been applied by the handle 1 (FIG. 5). The continued pulling proximally creates a retraction force, which tightens the coils of the coiled wire 220, as shown in FIG. 11C-5. The pulling proximally (in the direction of the arrow 11pp) overcomes the forces of the temporary frictional engagement and retention of the inner shaft 100 and the core wire 213, and causes the disengagement of the inner shaft 100 from the core wire 213. The coiled wire 220, behaves as a spring, and relaxes, releasing the energy stored in the spring, causing the tip element 21 to move distally, in the direction of the arrow 11F, as shown in FIG. 11C-6. The spring force (the released spring energy), for example, is in accordance with the forces described for FIGs. 4 and 4A above. This force is sufficient to allow the tip member 21 to safely penetrate and pass through coronary lesions.

FIG. 11D shows a handle 2000 for use with the guide wire 2-1011 of the FIG. 11 series. The handle 2000 includes sliders 2002, 2004. A distal slider 2002 allows for manual control of proximal (toward the proximal end PE of the guide wire 2-1011) and distal movement (toward the distal end DE of the guide wire 2-1011) of the inner shaft 100 (represented by the double headed arrow 2002a). The proximal side of the external shaft 20/26 is fixed to the body of the handle 2000, such that moving the distal slider 2002 causes relative motion between the inner shaft 100 and the external shaft 20/26. The proximal slider 2004 allows for adjustment (in the directions of the double headed arrow 2004a) of the distance between the sliders 2002, 2004, and accordingly, the compression of the spring 2006 between the sliders 2002, 2004. The spring 2006 and proximal slider 2004 function in the same manner as the spring 12 and knob 13 of the handle 1 of FIG. 5.

In another embodiment, the handle 1, illustrated in FIGs. 1 and 5, is removed by cutting the wire 2, as represented by the scissors 133 cutting along the broken line 133a. A PTCA balloon, catheter, other instrumentation, or the like can now be introduced over the wire 2, or the wire 2 can be exchanged with another wire, instrument or the like.

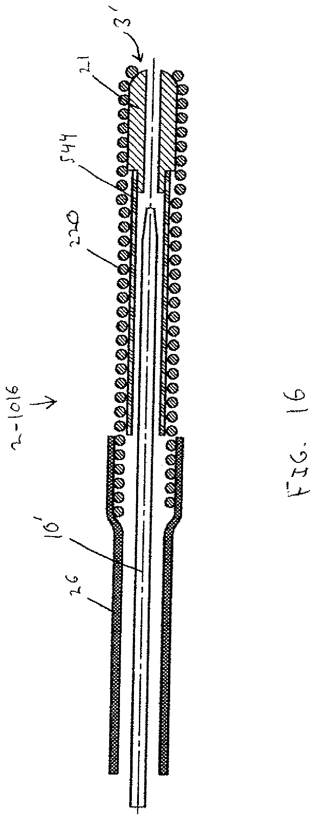

While the handle 1 of FIG. 5 is shown with the wire 2 of FIGs. 2, 3, 6 and 7, the handle 1 also operates the same for the guide wires 2-1012 (FIGs. 12 and 12A), 2-1013 (FIGs. 13 and 13A), 2-1016 (FIG. 16), respectively, as the spring 12 attaches to the inner shafts 108, 10, 10', and the gripping collar 25 attached to external shaft 20/26.

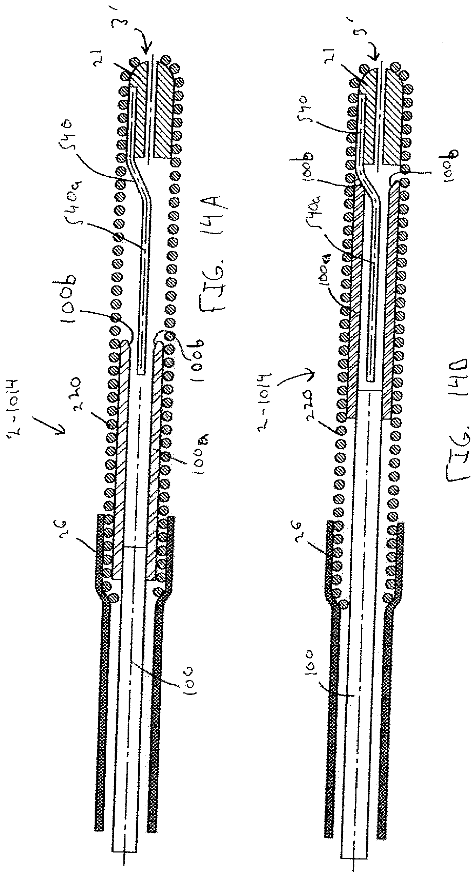

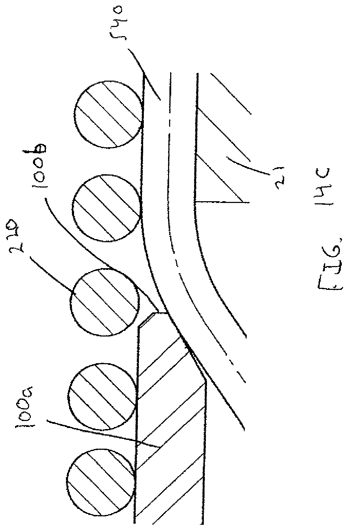

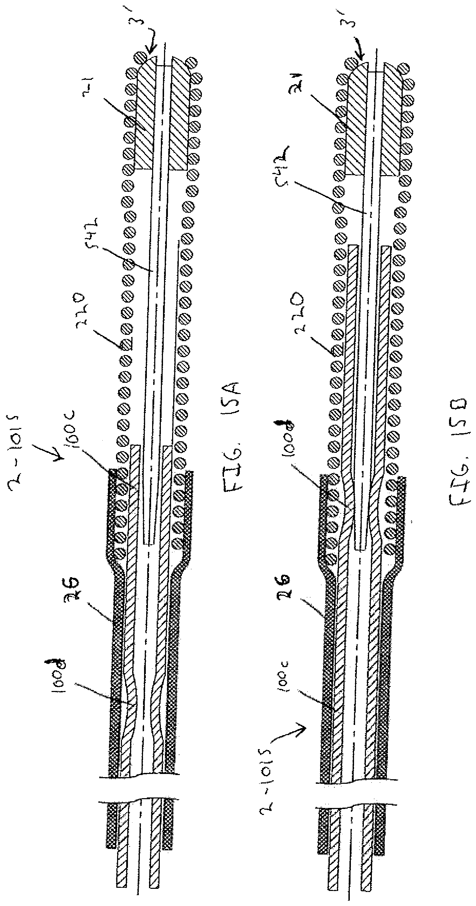

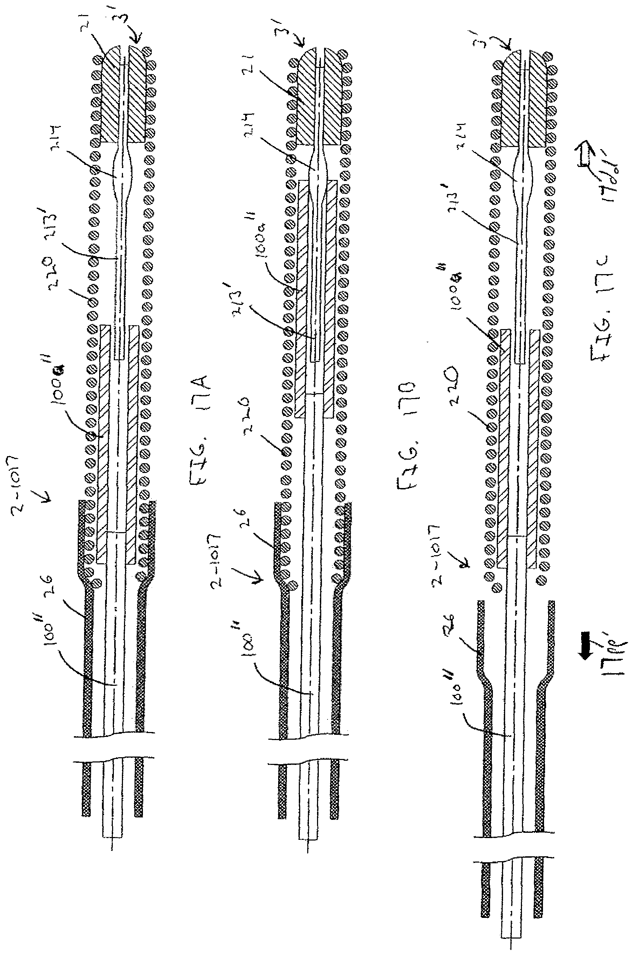

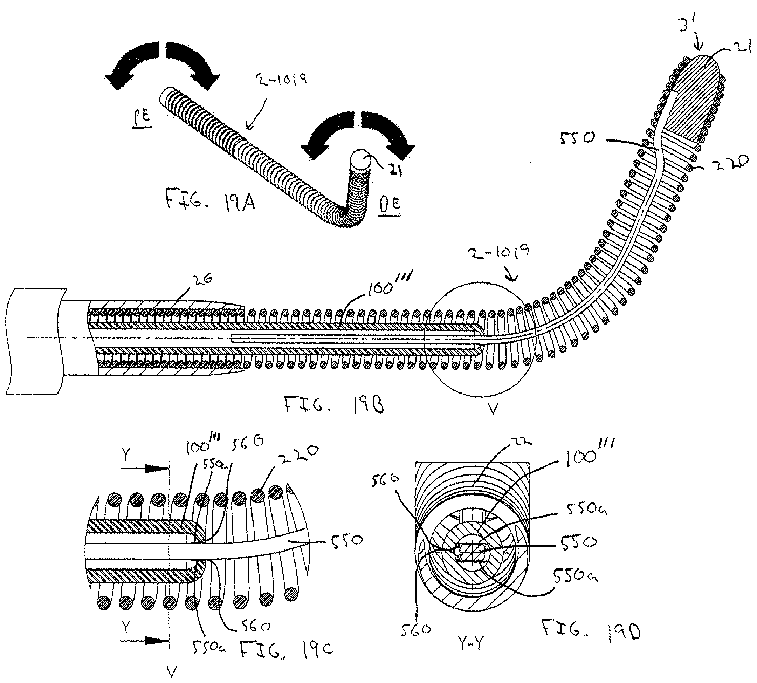

The handle 1 may be adapted for the spring 12 to connect to the inner shafts 100 (of guide wire 2-1010 of FIG. 10), 100 (of guide wire 2-1011 of the FIG. 11 series), 100 (of guide wire 2-1014 of the FIG. 14 series), 100c (of guide wire 2-1015 of the FIG. 15 series), 100" (of guide wire 2-1017 of the FIG. 17 series), and 100''' (of guide wire 2-1019 of the FIG. 19 series). In these guide wires 2-1010, 2-1011, 2-1014, 2-1015, 2-1016, 2-1017, and 2-1019, the external shafts 20/26 are attached to the gripping collar 25, in accordance with FIG. 5.

The guide wire 2-1013 of FIGs. 13 and 13A includes a thin safety wire 30 connected to the distal tip 21 of the external shaft 20. The safety wire 30 connected to the distal tip define the tip 3' of the guide wire 2-1013, in accordance with the tip 3 of the guide wire 2 of FIG. 1, and described above. The safety wire 30 can form a frictional engagement with the inner shaft 10, as described above. The frictional engagement is temporary, as shown in FIG. 13A, and will cause a penetration force in the distal direction when the engagement force is broken, as detailed above. In the case of a catastrophic scenario where the inner shaft 10, i.e., the wire forming the inner shaft 10, breaks down in the vessel, the thin safety wire 30 permits the removal of the fragments.

FIGs. 14A-14C show a guide wire 2-1014 with a side interlock. A bent wire 540 is attached to the tip member 21. The bent wire 540 and the tip member 21 define the tip 3' of the guide wire 2-1014, in accordance with the tip 3 of the guide wire 2 of FIG. 1, and described above. An inner shaft 100, having a distal sleeve 100a with tapered distal ends 100b, when moved distally, and telescopically over a proximal portion 540a of the bent wire 540, rests in a space between the wire 540 and the coiled portion 22, forming a temporary frictional engagement and temporary interlocking, between the distal sleeve 100a, and the bent wire 540 and the coiled portion 22, as shown in FIG. 14B, and in detail in FIG. 14C. The characteristics and behavior of this guide wire 2-1014 are, for example, in accordance with the guide wire 2-1011, as discussed above and shown in the FIG. 11 series..

FIGs. 15A and 15B show a guide wire 2-1015 with a proximal interlock. A core wire 542 is attached to the tip member 21. The core wire 542 and the tip member 21 define the tip 3' of the guide wire 2-1015, in accordance with the tip 3 of the guide wire 2 of FIG. 1, and described above. An inner shaft (similar to inner shaft 100 of FIGs. 14A and 14B, but not shown) includes a distal sleeve 100c with and inwardly tapered portion 100d. When the sleeve 100c is moved distally, and telescopically over the core wire 542, the movement terminates in a temporary frictional engagement and temporary interlocking of the sleeve 100c at the inwardly tapered portion 100d, with the core wire 542, as shown in FIG. 15B. The characteristics and behavior of this guide wire 2-1015 are, for example, in accordance with the guide wires 2-1011 (FIG. 11 series), and 2-1014 (FIG. 14 series), as discussed above.

FIG. 16 shows a guide wire 2-1016 having a tip member 21 from which an attached sleeve 544 extends proximally. The sleeve 544 telescopically receives a movable inner shaft 10' (similar to inner shaft 10 of FIGs. 13 and 13A as described above), movable by the handle 1 as described for FIGs. 13 and 13A above. The sleeve 544 serves as a safety mechanism, preventing the inner shaft 10' from protruding through the coiled wire 220 of the guide wire 2-1016.

FIGs. 17A-17C shows a guide wire 2-1017 having a tip member 21 from which an attached core wire 213', similar to core wire 213 of the guide wire 2-1011, as shown in the FIG. 11 series and described above. The tip member 21 and core wire 213' define a tip 3' of the guide wire 2-1017, in accordance with the tip 3 of the guide wire 2 of FIG. 1, and described above. An inner shaft 100"with a distal sleeve 100a", similar to inner shaft 100 of guide wire 2-1014, shown in FIGs. 14A-14C, and as described above, is moveable over the core wire 213', in a telescopic manner.

The sleeve 100a" is shown as disengaged from the core wire 213', with the coiled wire 220 being floppy, in FIG. 17A. In FIG. 17B, there is a frictional engagement and temporary interlock between the sleeve 100a" and the core wire 213', in accordance with that described for the guide wire 2-1011, and shown, for example, in the FIGs. 11A, 11C-1, 11C-2 and 11C-3. FIG. 17C shows a retraction of the inner shaft 100" (in accordance with the arrow 17pp') and disengagement of the sleeve 100a" from the core wire 213', with the tip member 21 advancing distally (in the direction of arrow 17dd') with an impact force as the coil wire 22 relaxes, releasing spring energy, as described for the guide wire 2-1011 of FIGs. 11C-5 and 11C-6, as detailed and shown above. The sleeve 100a" serves as a safety mechanism, preventing the core wire 213' from protruding through the coiled wire 220 of the guide wire 2-1017.

Additionally, in order to assure X-ray visualization, portions of the tip members 21, 21' of the guide wires 2-1010, 2-1011, 2-1012, 2-1013, 2-1014, 2-1015, 2-1016, 2-1017, and 2-1019 detailed above, and below (for guide wire 2-1019), for example, are coated with, or made of, radio opaque materials such as Gold, Platinum, or Tantalum. Alternately, for visualizing the motion between the inner 10, 100, 108 and outer or external 20/26 shafts, the tip of inner shafts may be coated or made of any of the aforementioned radio-opaque materials.

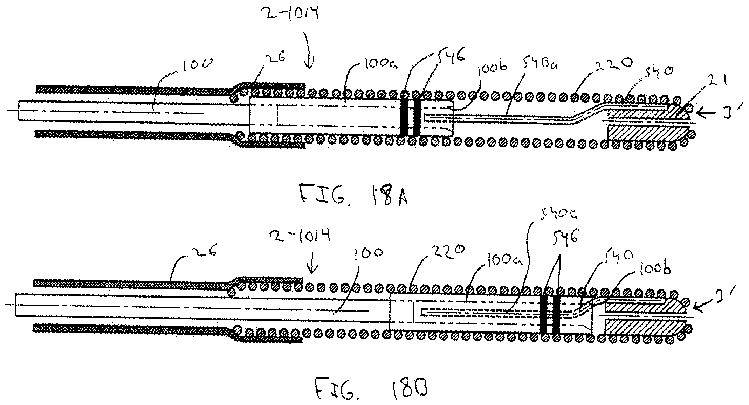

FIGs. 18A and 18B show the guide wire 2-1014, described above, and shown in FIGs. 14A-14C, with imaging markers 546, such as radio-opaque markers, as described above, on the distal end of the sleeve 100a of the inner shaft 100. In FIG. 18A, the sleeve 100a is disengaged from the tip member 21, and in FIG. 18B, the sleeve 100a has been moved distally into engagement (and temporary frictional interlocking) with the tip member 21.

FIGs. 19A-19D show a guide wire 2-1019, with a torque transmission system. In FIG. 19A the guide wire 2-1019 is shown with torque applied in the direction of the arrows at the proximal end PE, controlled by the handle 1 (FIG. 5), as detailed above, and the distal end DE, terminating at the tip member 21.

In FIG. 19B, the guide wire 2-1019 has a tip member 21 with a core wire 550 attached thereto, the tip member 21 and core wire 550 defining a 3' of the guide wire 2-101 1, in accordance with the tip 3 of the guide wire 2 of FIG. 1, and described above. The core wire 550 includes flattened oppositely disposed surfaces 550a, which are grippable by flattened lips 560 of an inner shaft 100'''. The inner shaft 100''' is moveable over and along the core wire 550 in a telescoping manner, as shown in FIGs. 19B, 19C (Detailed View V of FIG. 19B) and 19D. Upon torsion being applied to the inner shaft 100''', the flattened lips 560 grip the flattened surfaces 550a of the core wire, causing torsion (twisting) in the core wire 550, which is translated to the tip member 21.

The aforementioned tip members 21, 21' can be made of materials such as surgical grade metals, polymers and the like. The tip members 21, 21' may be coated with imaging markers, as detailed above.

**Claims**

1. A guidewire comprising:

   (a) a tubular member (20) including a proximal end and a distal end, the distal end including a coiled wire portion (22, 220);
   (b) a tip (3') including a tip member (21) associated with the distal end of the tubular member and a core wire (213, 213', 540, 542, 550) fixed to the tip member (21), the core wire (213) extending proximally from the tip member within the distal end of the tubular member; and
   (c) an inner shaft (100, 100', 100", 100''') extending along and displaceable longitudinally within the tubular member, the inner shaft engaging telescopically so as to be slidable over the core wire, thereby varying an overlap between the inner shaft and the coiled wire portion (22, 220),

   **characterized in that** the inner shaft (100) is configured to frictionally engage the core wire (213, 213', 540, 542, 550) so that longitudinal motion of the inner shaft (100) in a proximal direction within the tubular member core wire generates a corresponding proximal displacement of the tip (3), and wherein, when sufficient longitudinal force is applied in a proximal direction to overcome the frictional engagement, the inner shaft moves telescopically so as to be slidable over the core wire (213).

## Patentansprüche

1. Führungsdraht, umfassend:

   (a) ein röhrenförmiges Glied (20), das ein proximales und ein distales Ende aufweist, wobei das distale Ende einen Spiraldrahtabschnitt (22, 220) aufweist,
   (b) eine Spitze (3'), die ein Spitzenglied (21), das dem distalen Ende des röhrenförmigen Glieds zugeordnet ist, und einen am Spitzenglied (21) befestigten Kerndraht (213, 213', 540, 542, 550) aufweist, wobei sich der Kerndraht (213) in dem distalen Ende des röhrenförmigen Glieds proximal von dem Spitzenglied erstreckt, und
   (c) einen inneren Schaft (100, 100', 100", 100'''), der sich entlang dem röhrenförmigen Glied erstreckt und in Längsrichtung darin verschiebbar ist, wobei der innere Schaft teleskopisch in Eingriff kommt, so dass er über den Kerndraht gleiten kann, wodurch eine Überlappung zwischen dem inneren Schaft und dem Spiraldrahtabschnitt (22, 220) variiert wird,

   **dadurch gekennzeichnet, dass** der innere Schaft (100) dazu ausgestaltet ist, den Kerndraht (213, 213', 540, 542, 550) in Reibeingriff zu nehmen, so dass eine Längsbewegung des inneren Schafts (100) in einer proximalen Richtung in dem Kerndraht des röhrenförmigen Glieds eine entsprechende proximale Verschiebung der Spitze (3) erzeugt, und wobei sich, wenn eine hinreichende Längskraft in einer proximalen Richtung ausgeübt wird, um den Reibeingriff zu überwinden, der innere Schaft teleskopisch bewegt, so dass er über den Kerndraht (213) gleiten kann.

## Revendications

1. Fil-guide comprenant:

   (a) un élément tubulaire (20) comprenant une extrémité proximale et une extrémité distale, l'extrémité distale comprenant une partie de fil enroulée (22, 220);
   (b) une pointe (3') comprenant un élément de pointe (21) associé à l'extrémité distale de l'élément tubulaire et un fil central (213, 213', 540, 542, 550) fixé à l'élément de pointe (21), le fil central (213) s'étendant de manière proximale depuis l'élément de pointe dans l'extrémité distale de l'élément tubulaire; et
   (c) un arbre intérieur (100, 100', 100", 100''') s'étendant le long de l'élément tubulaire et pouvant se déplacer longitudinalement à l'intérieur de celui-ci, l'arbre intérieur s'engageant de façon télescopique de manière à pouvoir glisser sur le fil central, variant ainsi un recouvrement entre l'arbre intérieur et la partie de fil enroulée (22, 220),

**caractérisé en ce que** l'arbre intérieur (100) est configuré pour venir en prise par friction avec le fil central (213, 213', 540, 542, 550) de sorte que le mouvement longitudinal de l'arbre intérieur (100) dans une direction proximale à l'intérieur du fil central de l'élément tubulaire génère un déplacement proximal correspondant de la pointe (3), et dans lequel, quand une force longitudinale suffisante est appliquée dans une direction proximale pour surmonter la prise par friction, l'arbre intérieur se déplace de façon télescopique afin d'être mobile par-dessus le fil central (213).

FIG 1

210'

220

2

11

10

20

23

21

22

X-X

FIG 2

FIG 3

FIG 4A

FIG 4

FIG 5

FIG 6

FIG 7

FIG 8

FIG 9

FIG 10

FIG. 11

FIG. 11A

FIG. 110-1

FIG 110-2

FIG. 11B-3

EP 2 812 062 B1

FIG. 11C-1

FIG. 11C-2

FIG. 11C-3

EP 2 812 062 B1

26  100  2-1011  220  213  214  21  3'

11PP

FIG. 11C-4

2-1011

26  220  100  214  213  21  3'

11PP

FIG. 11C-5

2-1011

26

100

213  220  214  21

3'

FIG. 11C-6

11PP

11F

FIG. 11D

FIG. 12

FIG. 12A

EP 2 812 062 B1

FIG 13

FIG 13A

FIG. 14A

FIG. 14B

EP 2 812 062 B1

FIG. 14C

2 - 1015

100d

26

100c

220

542

2-1

3'

FIG. 15A

2 - 1015

100c

26

100d

220

542

2-1

3'

FIG. 15B

FIG. 16

EP 2 812 062 B1

FIG. 17A

FIG. 17B

FIG. 17C

FIG. 18A

FIG. 18B

36

FIG. 19A

FIG. 19B

FIG. 19C

FIG. 19D

EP 2 812 062 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61597783 A
- US 5040543 A, Badera
- US 5605162 A, Mirzaee

**Non-patent literature cited in the description**

- **HEE-YEOL KIM.** Percutaneous Recanalization of Coronary Chronic Total Occlusions: Current Devices and Specialized Wire Crossing Techniques. *Korean Circ J,* 2010, vol. 40, 209-215